# EUROPEAN PATENT APPLICATION

(11) **EP 3 111 953 A1**
(43) Date of publication of application: **04.01.2017**
(21) Application number: 15174810.0
(22) Date of filing: 01.07.2015
(51) Int. Cl.: A61K 39/145, C07K 16/10

(54) **WHOLE-INACTIVATED INFLUENZA VIRUS AS AN ADJUVANT FOR PEPTIDE ANTIGENS**

(71) Applicant: De Staat der Nederlanden, vert. door de minister Van VWS, Ministerie van Volksgezondheid, Welzijn en Sport, 2500 EJ Den Haag (NL)
(72) Inventor: Amorij, Jean-Pierre, 1391 SZ Abcoude (NL); Kersten, Gideon Frank Anne, 3514 HT Utrecht (NL); Soema, Peter Christiaan, 2561 EX Utrecht (NL)
(74) Representative: Nederlandsch Octrooibureau

(57) **Abstract**

The present invention relates to the use of Whole inactivated influenza virus (WIV) for use as adjuvant when raising an immune response to an antigen. The WIV is preferably used as adjuvant for raising a cellular immune response such as a CTL response against the antigen, preferably using a peptide comprising an epitope of the antigen as immunogen. The antigen can be an antigen of a tumor or of an infectious agent, e.g. a virus such as influenza virus. The WIV is particularly suitable for raising a cellular immune response against conserved, preferably non-surface exposed, epitopes of viruses such as influenza.

SEQUENCE LISTING

<110> De Staat der Nederlanden, vert. door de minister van VWS,
Ministerie van Volksgezondheid, Welzijn en Sport

<120> Whole-inactivated influenza virus as an adjuvant for peptide
antigens

<130> P6055023EP

<160> 3

<170> PatentIn version 3.3

<210> 1
<211> 9
<212> PRT
<213> Artificial

<220>
<223> Synthetic peptide

<400> 1

Gly Ile Leu Gly Phe Val Phe Thr Leu
1               5

<210> 2
<211> 9
<212> PRT
<213> Artificial

<220>
<223> Synthetic peptide

<400> 2

Phe Met Tyr Ser Asp Phe His Phe Ile
1               5

<210> 3
<211> 9
<212> PRT
<213> Artificial

<220>
<223> Synthetic peptide

<400> 3

Asn Met Leu Ser Thr Val Leu Gly Val
1               5

## Description

### Field of the invention

The present invention relates to the field of medicine, in particular the fields of immunology, vaccinology, medical microbiology and virology. More specifically, the invention relates to whole-inactivated influenza virus for use as adjuvant for raising an immune response against peptide antigen.

### Background of the invention

Current seasonal influenza vaccines mediate their protective effect mainly through the induction of virus-specific neutralizing antibodies. These antibodies are directed against the influenza surface proteins hemagglutinin (HA) and neuraminidase (NA). However, these proteins constantly undergo changes due to antigenic shifts and drifts, which can alter the antibody binding sites. These antigenic changes impair the neutralizing ability of antibodies induced by vaccines, rendering these vaccines ineffective. Therefore, additional immune responses such as cellular responses against influenza need to be induced to increase vaccine effectiveness.

Cellular immune responses represent a potential alternative to antibody-mediated immune responses. Recently, Sridhar et al. found cellular correlates of protection against pandemic influenza in the absence of neutralizing antibodies [1], which confirmed that cellular responses against influenza can indeed be effective. Cellular immune responses such as cytotoxic T-cells (CTLs) can effectively clear virus-infected host cells, thereby inhibiting viral replication and spread. Unlike most vaccine-induced antibodies, these CTLs recognize epitopes located on internal influenza proteins, which are conserved by many influenza strains. Due to the conserved nature of these epitopes, cellular responses directed against these epitopes are potentially cross-reactive. Short linear peptides representing these epitopes are therefore attractive antigens for the development of cross-reactive influenza vaccines.

Peptide antigens as such however suffer from low immunogenicity, due to the lack of peptide delivery to antigen presenting cells (APCs) and the absence of pathogen-associated molecular patterns (PAMPs) or adjuvants to activate the APCs. Delivery of the peptide antigen to the cytoplasm of APCs is considered to be crucial for proper processing and subsequent presentation on MHC class I molecules, while activation of the APCs is important for licensing of naive CD8+ T-cells [2].

Formulation of peptide antigens with an appropriate adjuvant (which can be a delivery system or an immunopotentiator [3]) is thus crucial to induce an cellular immune response against the peptide antigen. Water-in-oil emulsions such as Montanide are effective adjuvants for peptides, but are associated with severe adverse events such as lesion formation at the site of injection [4]. Thus, alternative adjuvants for peptide antigens are highly sought after. Particulate adjuvant systems such as liposomes or virosomes formulated with influenza peptides derived from internal proteins have proven to be effective for the induction of peptide-specific CTLs [5, 6], especially in combination with Toll-like receptor (TLR) agonists [7, 8]. However, the formulation of peptides into these delivery systems can be complicated, which can result in inefficient encapsulation rates. Adjuvants that can be directly admixed with peptide antigens would therefore be preferable.

Whole inactivated influenza virus (WIV) possesses an innate adjuvant capability in the form of influenza viral ssRNA, which is a potent TLR7 agonist [9]. However, WIV was never previously used or shown to be effective as an adjuvant for peptide antigens.

WO 2012/100302 discloses vaccine compositions comprising gamma-irradiated influenza viruses with additional immunogen(s), wherein the additional immunogen(s) are capable of stimulating the immune response against agent(s) causative of a target disease or condition, preferably a secondary infection or condition associated with influenza infection.

There is however still a need in the art for safe adjuvants that can easily be produced and that are effective as adjuvant in stimulating immune responses against low immunogenic antigens, particularly peptides requiring a cellular immune response.

### Summary of the invention

In a first embodiment, the invention relates to a whole inactivated influenza virus (WIV) for use as adjuvant in the prevention and/or treatment of a disease or a condition associated with an antigen. The treatment preferably comprises raising an immune response against the antigen by administration of the WIV and an immunogen comprising or encoding at least one epitope of the antigen. The WIV preferably is a chemically inactivated WIV, more preferably a WIV inactivated by at least one of formalin and β-propiolactone.

In a second embodiment, the WIV is for a use according to the first embodiment, wherein the disease or a condition is an infectious disease and/or a tumor, wherein the immunogen induces an immune response against an antigen of an agent causing the infectious disease and/or an antigen of the tumor.

In a third embodiment, the WIV is for a use according to the first or second embodiment, wherein the treatment comprises co-localized administration of the WIV and the immunogen, wherein preferably, the co-localized administration comprises administration at one or more sites of administration that drain to the same lymph node.

In a fourth embodiment, the WIV is for a use according to the third embodiment, wherein the co-localized administration comprises the administration of a single composition comprising both the WIV and the immunogen.

In a fifth embodiment, the WIV is for a use according to any one of the preceding embodiments, wherein the immune response is a cellular immune response, preferably a T-cell response comprising at least one of a CD8⁺ cytotoxic T-cell (CTL) response and a CD4⁺ T helper (Th) cell response.

In a sixth embodiment, the WIV is for a use according to any one of the preceding embodiments, wherein the immunogen is selected from the group consisting of a protein, a peptide, a chemically modified protein or peptide, a protein or peptide comprising non-proteogenic amino acids, a polysaccharide, a whole bacterial cell, a membrane vesicle, an outer membrane vesicle, a virion, a virus-like particle, a nucleic acid and conjugates and combinations of thereof.

In a seventh embodiment, the WIV is for a use according to the sixth embodiment, wherein the antigen is a proteinaceous antigen and the immunogen is a peptide comprising at least one epitope of the antigen.

In a eighth embodiment, the WIV is for a use according to the seventh embodiment, wherein the peptide comprise at least one of an HLA class I restricted CTL epitope and an HLA class II restricted Th epitope.

In a ninth embodiment, the WIV is for a use according to the eighth embodiment, wherein the peptide comprises a CTL epitope of a virus, which epitope is conserved between different strains of the virus, which epitope preferably is not exposed at the surface of the virus and wherein more preferably, the virus is influenza.

In a tenth embodiment, the WIV is for a use according to the ninth embodiment, wherein the peptide comprises a conserved CTL epitope located in an influenza protein selected from the group consisting of the matrix protein (M1), the membrane protein (M2), one RNA polymerase complex proteins (PB1, PB2 and PA) and nucleoprotein (NP).

In an eleventh embodiment, the WIV is for a use according to any one of the sixth to tenth embodiments, wherein the peptide is at least one of: a) a peptide having a length of between 6 and 100 amino acids; and, b) a peptide comprising one or more non-proteogenic amino acids.

In a twelfth embodiment, the WIV is for a use according to any one of the preceding embodiments, wherein the WIV is a WIV vaccines, preferably a WIV vaccine comprising interpandemic annual or/and seasonal influenza strains.

In a thirteenth embodiment, the WIV is for a use according to any one of the preceding embodiments, wherein the WIV is administered in a dosage of between 0.5 and 500 µg hemagglutinin per dose.

In a fourteenth embodiment, the invention pertains to a pharmaceutical composition comprising a WIV as defined in any one of the preceding embodiments and an immunogen as defined in any one of the preceding embodiments, wherein preferably, the immunogen is present as an entity separate from the WIV.

In a fifteenth embodiment, the invention pertains to a kit of parts comprising a first container comprising a WIV as defined in any one of the preceding embodiments and at least a second container comprising an immunogen as defined in any one of the preceding embodiments.

### Description of the invention

### Definitions

The term "immune response" as used herein refers to the production of antibodies and/or cells (such as T lymphocytes) that are directed against, and/or assist in the decomposition and/or inhibition of, a particular antigenic entity, carrying and/or expressing or presenting antigens and/or antigenic epitopes at its surface. The phrases "an effective immunoprotective response", "immunoprotection", and like terms, for purposes of the present invention, mean an immune response that is directed against one or more antigenic epitopes of a pathogen, a pathogen-infected cell or a cancer cell so as to protect against infection by the pathogen or against cancer in a vaccinated subject. For purposes of the present invention, protection against infection by a pathogen or protection against cancer includes not only the absolute prevention of infection or cancer, but also any detectable reduction in the degree or rate of infection by a pathogen or of the cancer, or any detectable reduction in the severity of the disease or any symptom or condition resulting from infection by the pathogen or cancer in the vaccinated subject, for example as compared to an unvaccinated infected subject. An effective immunoprotective response in the case of cancer also includes clearing up the cancer cells, thereby reducing the size of cancer or even abolishing the cancer. Vaccination in order to achieve this is also called therapeutic vaccination. Alternatively, an effective immunoprotective response can be induced in subjects that have not previously been infected with the pathogen and/or are not infected with the pathogen or do not yet suffer from cancer at the time of vaccination, such vaccination can be referred to as prophylactic vaccination.

According to the present invention, the general use herein of the term "antigen" refers to any molecule that binds specifically to an antibody. The term also refers to any molecule or molecular fragment that can be bound by an MHC molecule and presented to a T-cell receptor. Antigens can be e.g. proteinaceous molecules, i.e. polyaminoacid sequences, optionally comprising non-protein groups such as carbohydrate moieties and/or lipid moieties or antigens can be e.g. molecules that are not proteinaceous such as carbohydrates. An antigen can be e.g. any portion of a protein (peptide, partial protein, full-length protein), wherein the protein is naturally occurring or synthetically derived, a cellular composition (whole cell, cell lysate or disrupted cells), an organism (whole organism, lysate or disrupted cells) or a carbohydrate or other molecule, or a portion thereof, that is able to elicit an antigen-specific immune response (humoral and/or cellular immune response) in a particular subject, which immune response preferably is measurable via an assay or method.

The term "antigen" is herein understood as a structural substance which serves as a target for the receptors of an adaptive immune response. An antigen thus serves as target for a TCR (T-cell receptor) or a BCR (B-cell receptor) or the secreted form of a BCR, i.e. an antibody. The antigen can thus be a protein, peptide, carbohydrate or other hapten that is usually part of a larger structure, such as e.g. a cell or a virion. The antigen may originate from within the body ("self') or from the external environment ("non-self'). The immune system is usually non-reactive against "self" antigens under normal conditions due to negative selection of T cells in the thymus and is supposed to identify and attack only "non-self" invaders from the outside world or modified/harmful substances present in the body under e.g. disease conditions. Antigens structures that are the target of a cellular immune response are presented by antigen presenting cells (APC) in the form of processed antigenic peptides to the T cells of the adaptive immune system via a histocompatibility molecule. Depending on the antigen presented and the type of the histocompatibility molecule, several types of T cells can become activated. For T-Cell Receptor (TCR) recognition, the antigen is processed into small peptide fragments inside the cell and presented to a T-cell receptor by major histocompatibility complex (MHC).

The term "immunogen" is used herein to describe an entity that comprises or encodes at least one epitope of an antigen such that when administered to a subject, preferably together with an appropriate adjuvant, elicits a specific humoral and/or cellular immune response in the subject against the epitope and antigen comprising the epitope. An immunogen can be identical to the antigen or at least comprises a part of the antigen, e.g. a part comprising an epitope of the antigen. Therefore, to vaccinate a subject against a particular antigen means, in one embodiment, that an immune response is elicited against the antigen or immunogenic portion thereof, as a result of administration of an immunogen comprising at least one epitope of the antigen. Vaccination preferably results in a protective or therapeutic effect, wherein subsequent exposure to the antigen (or a source of the antigen) elicits an immune response against the antigen (or source) that reduces or prevents a disease or condition in the subject. The concept of vaccination is well-known in the art. The immune response that is elicited by administration of a prophylactic or therapeutic composition of the present invention can be any detectable change in any facet of the immune status (e.g., cellular response, humoral response, cytokine production), as compared to in the absence of the administration of the vaccine.

An "epitope" is defined herein as a single immunogenic site within a given antigen that is sufficient to elicit an immune response in a subject. Those of skill in the art will recognize that T cell epitopes are different in size and composition from B cell epitopes, and that T cell epitopes presented through the Class I MHC pathway differ from epitopes presented through the Class II MHC pathway. Epitopes can be linear sequences or conformational epitopes (conserved binding regions) depending on the type of immune response. An antigen can be as small as a single epitope, or larger, and can include multiple epitopes. As such, the size of an antigen can be as small as about 5-12 amino acids (e.g., a peptide) and as large as: a full length protein, including multimeric proteins, protein complexes, virions, particles, whole cells, whole microorganisms, or portions thereof (e.g., lysates of whole cells or extracts of microorganisms).

As used herein, the term "antibody" means an immunoglobulin molecule or a fragment of an immunoglobulin molecule having the ability to specifically bind to a particular (epitope of an) antigen. Antibodies are well known to those of ordinary skill in the science of immunology.

The term "antibody mediated antigen-targeting" is used herein to indicate an antigen delivery system that uses antibody (such as e.g. IgG) mediated antigen targeting via Fc receptors that are expressed on the cell surface of APC, such as for example DC and macrophages. By making use of antigen-specific antibodies, complexes of these antibodies with the protein antigen can be formed in vitro but also in vivo. These complexes (so-called immune complexes (IC)) can be bound by the APC via these Fc receptors, subsequently taken up, and the antigen will then be processed and a peptide derived thereof will be presented to specific T cells. It is important that in antibody mediated antigen-targeting IC will also activate the APC and that leads to optimal stimulation of the specific T cells (Schuurhuis et al., J Immunol. 168, 2240-2246, 2002). Thus, in antibody mediated antigen-targeting, specific antibodies circulating in human beings are used to selectively target antigens to stimulate the cellular immune system.

The term "peptide" as used herein is defined as a chain of amino acid residues, usually having a defined sequence. As used herein the term peptide is interchangeable with the terms "polypeptide" and "protein". In the context of the present invention, the term "peptide" is defined as being any peptide or protein comprising at least two amino acids linked by a modified or unmodified peptide bond. The term "peptide" refers to short-chain molecules such as oligopeptides or oligomers or to long-chain molecules such as proteins. A protein/peptide can be linear, branched or cyclic. The peptide can include D amino acids, L amino acids, or a combination thereof. The peptide can include non-proteogenic amino acids. A peptide according to the present invention can comprise modified amino acids. Thus, the peptide of the present invention can also be modified by natural processes such as post-transcriptional modifications or by a chemical process. Some examples of these modifications are: acetylation, acylation, ADP-ribosylation, amidation, covalent bonding with flavine, covalent bonding with a heme, covalent bonding with a nucleotide or a nucleotide derivative, covalent bonding to a modified or unmodified carbohydrate moiety, bonding with a lipid or a lipid derivative, covalent bonding with a phosphotidylinositol, cross-linking, cyclization, disulfide bond formation, demethylation, cysteine molecule formation, pyroglutamate formation, formylation, gamma-carboxylation, hydroxylation, iodination, methylation, oxidation, phosphorylation, racemization, hydroxylation, etc. Thus, any modification of the peptide which does not have the effect of eliminating the immunogenicity of the peptide, is covered within the scope of the present invention.

The term "sequence identity" is herein defined as a relationship between two or more amino acid (peptide or protein) sequences or two or more nucleic acid (polynucleotide) sequences, as determined by comparing the sequences. Usually, sequence identities or similarities are compared over the whole length of the sequences compared. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one peptide to the sequence of a second peptide. "Identity" and "similarity" can be readily calculated by various methods, known to those skilled in the art. In a preferred embodiment, sequence identity is determined by comparing the whole length of the sequences as identified herein. Preferred methods to determine identity are designed to give the largest match between the sequences tested. Methods to determine identity and similarity are codified in publicly available computer programs. Preferred computer program methods to determine identity and similarity between two sequences include e.g. the BestFit, BLASTP, BLASTN, and FASTA (Altschul, S. F. et al., J. Mol. Biol. 215:403-410 (1990), publicly available from NCBI and other sources (BLAST Manual, Altschul, S., et al., NCBI NLM NIH Bethesda, MD 20894). A most preferred algorithm used is EMBOSS (http://www.ebi.ac.uk/emboss/align). Preferred parameters for amino acid sequences comparison using EMBOSS are gap open 10.0, gap extend 0.5, Blosum 62 matrix. Preferred parameters for nucleic acid sequences comparison using EMBOSS are gap open 10.0, gap extend 0.5, DNA full matrix (DNA identity matrix). Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is, alanine, valine, leucine, and isoleucine; a group of amino acids having aliphatic-hydroxyl side chains is serine and threonine; a group of amino acids having amide-containing side chains is asparagine and glutamine; a group of amino acids having acidic side chains is aspartic acid and glutamic acid; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred amino acid substitutions are substitution of an amino acid with an amino acid from the same conservative amino acid group. These conservative amino acid groups are: alanine-valine-leucine-isoleucine-methionine; phenylalanine-tyrosine-tryptophane; lysine-arginine-histidine; asparagine-glutamine; aspartic acid and glutamic acid; serine-threonine; glycine-proline. In this particular case another conservative amino acid substitution group is lysine-formyllysine

The terms "tumour" or "cancer" in a subject refers to the presence of cells possessing characteristics such as atypical growth or morphology, including uncontrolled proliferation, immortality, metastatic potential, rapid growth and proliferation rate, and certain characteristic morphological features. Often, cancer cells will be in the form of a tumour, but such cells may also exist in isolation from one another within a subject. "Tumour" includes both benign and malignant neoplasms.

In the context of the invention, a "patient" or "subject" may be an animal (including humans). Preferably, a patient or subject is a human being.

### Detailed description of the invention

The present invention relates to the use of whole inactivated influenza virus (WIV) as adjuvant. The present inventors have surprisingly discovered that the addition of WIV to peptide antigens promotes the induction of antigen-specific immune responses, in particular peptide antigen-specific T-cell responses.

In a first aspect therefore, the invention pertains to WIV for use as adjuvant, or the invention pertains to a method wherein WIV is used as adjuvant. Preferably, WIV is used as an adjuvant in a treatment comprising raising an immune response against an antigen. Raising an immune response against an antigen is herein understood to comprise both inducing a novel immune response as well as enhancing a previously existing immune response. Preferably, the treatment comprises administering WIV and an immunogen comprising or encoding at least one epitope of the antigen. Thus, preferably, WIV is for use as adjuvant in the treatment of a disease or a condition associated with an antigen, wherein the treatment comprises raising an immune response against the antigen by administration of the WIV and an immunogen comprising or encoding at least one epitope of the antigen a peptide comprising at least one epitope of the antigen. More preferably, the antigen is a proteinaceous antigen and the immunogen is a peptide comprising at least one epitope of the antigen.

The WIV for use as adjuvant, preferably is a chemically inactivated WIV as herein defined below, more preferably the WIV is inactivated by at least one of formalin and β-propiolactone.

Treatment of a disease or a condition associated with a antigen is herein understood to comprise both treatment and prevention of the disease or condition, whereby prevention of the disease or condition preferably at least comprises prevention of pathology and/or symptoms associated with the disease or condition.

The whole inactivated influenza virus (WIV) for use as adjuvant in accordance with the present invention can be a preparation comprising whole influenza virus in inactive form as is well known in the art for its use as a vaccine. Thus preferably the WIV comprises whole influenza enveloped virions comprising one or more of hemagglutinin (HA), neuraminidase (NA), matrix protein (M1), membrane protein (M2), and a segmented single-stranded negative-sense RNA genome that forms ribonucleoprotien complexes with the viral RNA-dependent RNA polymerase complex (consisting of the transcriptase PB1, PB2 and PA) and nucleoprotein (NP) molecules. The WIV can be of a human influenza virus or an animal influenza virus selected from the group consisting of avian influenza virus, equine influenza virus, porcine (e.g. swine) influenza virus, feline influenza virus. Influenza virus is more particularly selected from strains A, B and C, preferably from strains A and B, of which A is most preferred.

The WIV or antigens thereof may be derived from interpandemic (annual or seasonal) influenza strains. Alternatively, the WIV or antigens thereof may be derived from influenza strains with the potential to cause a pandemic outbreak. E.g. influenza strains with a new hemagglutinin compared to the hemagglutinin in currently circulating strains, or influenza strains which are pathogenic in avian subjects and have the potential to be transmitted horizontally in the human population, or influenza strains which are pathogenic to humans. Depending on the particular season and on the nature of the antigen included in the vaccine, the WIV or antigens thereof may be derived from one or more of the following hemagglutinin subtypes: H1, H2, H3, H4, H5, H6, H7, H8, H9, H10, H11, H12, H13, H14, H15 and H16. Preferably, the influenza virus or antigens thereof are from H1, H2, H3, H5, H7 and H9 subtypes. Furthermore, the WIV or antigens thereof may be derived from one or more of the following neuraminidase subtypes: N1, N2, N3, N4, N5, N6, N7, N8 and N9. Furthermore the WIV may comprise any combination of hemagglutinin and neuraminidase subtypes. A WIV with a particular combination of H- and N-subtypes can be used individually or in any combination with one or more WIV having other combinations of H- and N-subtypes.

Method for the preparation of WIV vaccines are well known in the art (see e.g. WHO Technical Report Series, No. 927, 2005, Annex 3 and other documentation at http://www.who.int/biologicals/vaccines/influenza/en/). The whole influenza virions for WIV can be in embryonated eggs or in cell lines, such as e.g. Vero cells. For inactivation of the whole influenza virions various means are known in the art. E.g. chemical means for inactivating a virus include treatment with an effective amount of one or more of the following agents: detergents (e.g. Triton X-100), formaldehyde, formalin, beta-propiolactone, or merthiolate. Additional chemical means for inactivation may include treatment with methylene blue, psoralen, carboxyfullerene (C60) or a combination of any thereof. Other methods of viral inactivation are known in the art, such as for example binary ethylamine, acetyl ethyleneimine, and physical means such as heat (pasteurization), UV light or gamma irradiation. Preferably for inactivation of whole influenza virions formalin (40% v/v formaldehyde) or β-propiolactone (2-oxetanone) is used, the concentration by volume should not exceed 0.1% at any time during inactivation. In a preferred method for inactivating whole influenza virions for preparing WIV, the virions are treated for at least 18 hours with at least about 0.02% v/v formalin, preferably, at about 37°C.

An inactivated virus is herein understood to mean a viral preparation with preferably no detectable infectivity. Infectivity of preparations of inactivated virus for use in the present invention may be determined using methods well known in the art (see e.g. Goldstein and Tauraso, 1970, Appl. Microbiol. 19: 290-294). Generally, the infectivity of an inactivated preparation is compared to the infectivity of a corresponding non-treated and/or mock-treated viral preparation whereby the titer of the inactivated viral preparation is at least 6, 7, 8, 9, or 10 orders of magnitude lower than the titer of the non-treated and/or mock-treated viral preparation. Preferably in the inactivated preparation no viral infectivity can be detected.

The WIV for use as adjuvant can be a fusion inactivated WIV, or a WIV having reduced membrane fusion activity. Membrane fusion activity of WIV can be reduced or eliminated by incubation at acidic pH, e.g. at pH 4.5, e.g. for at least 15 min. at 37 °C. Membrane fusion capacity can be determined by a hemolysis assay as described previously [8]. A WIV with reduced membrane fusion activity for use in the present invention preferably has no more than 75, 50, 25, 20, 10 or 5% of the membrane fusion activity of a corresponding untreated WIV, as can be assayed in a hemolysis assay.

In accordance with the invention, the WIV is used as adjuvant. An adjuvant is herein understood to be an entity, that, when administered in combination with an antigen to a human or an animal subject to raise an immune response against the antigen in the subject, stimulates the immune system, thereby provoking, enhancing or facilitating the immune response against the antigen, preferably without necessarily generating a specific immune response to the adjuvant itself. A preferred adjuvant enhances the immune response against a given antigen by at least a factor of 1.5, 2, 2.5, 5, 10 or 20, as compared to the immune response generated against the antigen under the same conditions but in the absence of the adjuvant. Tests for determining the statistical average enhancement of the immune response against a given antigen as produced by an adjuvant in a group of animal or human subjects over a corresponding control group are available in the art. The adjuvant preferably is capable of enhancing the immune response against at least two different antigens.

In accordance with the invention, the WIV is for use as adjuvant for raising an enhanced immune response against an antigen, which immune response can be B-cell (i.e. humoral) response and/or a T-cell (i.e. cellular) response. The immune response can be a B-cell response, i.e. involving the production of one or more antibodies specifically directed against the antigen. The antibody can be an IgA, IgM or IgG antibody, preferably an IgG2a and/or an IgG1 antibody.

However, preferably, the WIV is for use as adjuvant for raising an enhanced cellular immune response against an antigen, i.e. a T-cell response. The T-cell response preferably comprises at least one of a CD8⁺ cytotoxic T-cell response and a CD4⁺ T helper cell response against the antigen. The T helper cell response preferably is a Th1 response, a Th2 response or a balanced Th2/Th1 response. The skilled person knows that depending on the disease, a B and/or T cell response may be required to be induced for treating and/or preventing the disease.

Antigen specific immune responses and the enhancement thereof by the use of WIV as adjuvant in accordance with the invention can be determined by mean and methods well known in the art. A humoral response can e.g. be determined by a standard immunoassays for determining antibody levels in serum, including e.g. Western blot, immunoblot, enzyme-linked immunosorbant assay (ELISA), enzyme-linked immunosorbent spot (ELISPOT) or FluoroSpot assay, radioimmunoassay (RIA), immunoprecipitation, surface plasmon resonance and the like. Similarly, standard assays are known in the art for determining antigen-specific cellular immune responses. These assays include e.g. assays that determine the proliferation of antigen-specific T lymphocytes (e.g. ³H-thymidine incorporation assays or CFSE dilution assays), assays that determine the lytic activity of antigen-specific T lymphocytes, e.g. cytotoxic T-cells (⁵¹Chromium release assay, flow cytometric methods, e.g. detection CD107a expression or fluorescent antigen transfected target (FATT) cell cytotoxic T-cell assays), and assays that determine cytokine production by cytotoxic T-cells as marker for antigen-specific T-cell activity (e.g. Elispot or flow cytometric methods, e.g. as described in WO2011/049448).

In accordance with the invention, the WIV as adjuvant is used in a treatment which comprises the co-localized administration of the WIV and the immunogen. The co-localized administration of the WIV and the immunogen preferably comprises administration at one or more sites of administration (on the subject) that drain to the same lymph node. In one embodiment the co-localized administration of the WIV and the immunogen comprises the administration of a composition comprising the WIV and the immunogen, e.g. a mixture comprising at least the WIV and the immunogen. The WIV and the immunogen can be administered by parenteral and or by mucosal routes. Parenteral administration can be e.g. by injection or infusion by intravenous, subcutaneous, intraperitoneal, intramuscular, intraarterial or intralesional route. A preferred administration mode is subcutaneous. Alternatively, mucosal administration can be by oral, rectal, vaginal or intranasal route, e.g. by inhalation.

In the treatment, the WIV is preferably administered in a dosage of between 0.5 and 500 µg hemagglutinin per dose. The WIV is preferably administered in a dosage of at least 0.5, 1, 2, 4, or 8 µg hemagglutinin per dose, and preferably no more than 30, 50, 100, 200 or 500 µg hemagglutinin per dose. More preferably in the treatment the WIV is administered in a dosage of between 5 and 60 µg hemagglutinin per dose, most preferably in the treatment the WIV is administered in a dosage of between 10 and 20 µg hemagglutinin per dose, e.g. around 15 µg hemagglutinin per dose, the standard dose for WIV vaccines.

Also preferably in the treatment, the one or more immunogens is administered in a dosage of between 10 µg and 10 mg per dose, whereby preferably the one or more immunogens are one or more immunogenic peptides. More preferably, the one or more immunogens or peptides are administered in a dosage of at least 10, 20, 40, 100, 200, 400, 1000 µg per dose and preferably no more than 10, 5, 2, 1.5, or 1.2 mg per dose. Most preferably in the treatment, at least one of the WIV and the immunogen/peptide is administered at a dosage of at least 200, 400, 1000 µg per dose.

In a preferred embodiment, wherein the WIV is used (in a method) as adjuvant in the treatment of a disease or a condition associated with an antigen, the disease or a condition is preferably is at least one of an infectious disease and a tumor, and the immunogen preferably elicits an immune response against an antigen of an agent causing the infectious disease or against an antigen of the tumor. Preferably the antigen is a proteinaceous antigen and the immunogen is a peptide comprising at least one epitope of the antigen.

The immunogen in the treatment comprising the use of the WIV adjuvant according to the invention preferably is selected from the group consisting of a protein, a peptide, a chemically modified protein or peptide, a protein or peptide comprising non-proteogenic amino acids, a polysaccharide, a whole bacterial cell, a membrane vesicle, an outer membrane vesicle, a virion, a virus-like particle, a nucleic acid and conjugates and combinations of thereof. The immunogen can thus be any component usually used in vaccines, including e.g. subunit vaccines, epitope vaccines modified proteins such as toxoids, native or modified antigens, carbohydrates such as (capsular) polysaccharides and lipopolysaccharides. Specifically includes as immunogens are nucleic acids encoding at least one epitopes of a proteinaceous antigen, which nucleic acids are capable of being expressed in the subject to be vaccinated and to which the WIV and the nucleic acid immunogen are administered.

The WIV and the immunogen are at least two separate entities that are both administered in the treatment. Nevertheless, in some embodiments these separate entities may be covalently or otherwise linked together. The immunogen may e.g. be conjugated to the WIV.

In one embodiment of the invention the immunogen is a peptide, preferably peptide comprising at least one epitope of the antigen, referred to herein as an immunogenic peptide. An immunogenic peptide is used for inducing and/or enhancing an immune response, preferably a T cell response, elicited by a T cell epitope within a immunogenic peptide. A preferred T-cell response induced and/or enhanced by an immunogenic peptide comprise at least one of an HLA class I restricted CTL response and an HLA class II restricted Th response. More preferably the T-cell response consists of both an HLA class I restricted CTL response and simultaneously an HLA class II restricted Th response, and may be advantageously accompanied by a B-cell response. In one embodiment, an immunogenic peptide comprises at least a HLA class I epitope and a HLA class II epitope, and/or the at least a HLA class I epitope and a HLA class II epitope are administered together on at least two different immunogenic peptides.

In one embodiment, the immunogenic peptides comprises an amino acid sequence that does not naturally occur, i.e. that does not exist in nature but that is the result of human intervention and/or design. In this embodiment, an immunogenic peptide will comprise one or more epitopes, e.g. as defined above, whereby such an epitope preferably consists of naturally amino acid sequences of antigens from an infectious agent and/or tumor. However, at least one amino acid sequence in the immunogenic peptide that flanks at least one epitope in the immunogenic peptide and/or that links two epitopes in the immunogenic peptide is not from the (naturally occurring or wild type) antigen from which the epitope(s) are derived and/or the linking/flanking amino acid sequence is from other locations within the antigen that are not contiguous with the epitope they flank.

In one embodiment the immunogenic peptide is a synthetic peptide. The use of relatively short peptides is highly preferred for medical purposes as these can be synthesized in vitro efficiently, which is not possible or uneconomical for native proteins larger than about 100 amino acids. Chemical synthesis of peptides is routine practice and various suitable methods are known to the skilled person. The length of a immunogenic peptide and/or a synthetic immunogenic peptide preferably is at least 6, 7, 8, 9, 10, 11, 12, 13, 14, 15, 16, 17, 18, 19, 20, 21, 22, 25, 27, 30, 33, 35, 40 or 45 amino acids and preferably no more than 100, 80, 60, 50 amino acids. The immunogenic peptide can include D amino acids, L amino acids, α- and β-amino acids and a combinations thereof. The immunogenic peptide can also include non-proteogenic amino acids, including e.g. homocysteine, phosphoserine, phosphothreonine, phosphotyrosine, hydroxyproline, γ-carboxyglutamate, hippuric acid, octahydroindole-2-carboxylic acid, statine, 1,2,3,4,-tetrahydroisoquinoline-3-carboxylic acid, penicillamine (3-mercapto-D-valine), ornithine (Orn), citruline, α-methyl-alanine, para-benzoylphenylalanine, para-aminophenylalanine, p-fluorophenylalanine, phenylglycine, propargylglycine, N-methylglycins (sarcosine, Sar), and tert-butylglycine, diaminobutyric acid, 7-hydroxy-tetrahydroisoquinoline carboxylic acid, naphthylalanine, biphenylalanine, cyclohexylalanine, amino-isobutyric acid (Aib), norvaline, norleucine (Nle), tert-leucine, tetrahydroisoquinoline carboxylic acid, pipecolic acid, phenylglycine, homophenylalanine, cyclohexylglycine, dehydroleucine, 2,2-diethylglycine, 1-amino-1-cyclopentanecarboxylic acid, 1-amino-1-cyclohexanecarboxylic acid, amino-benzoic acid, amino-naphthoic acid, γ-aminobutyric acid, difluorophenylalanine, nipecotic acid, N-α-imidazole acetic acid (IMA), thienyl-alanine, t-butylglycine, desamino-Tyr, aminovaleric acid (Ava), pyroglutaminic acid (<Glu), α-aminoisobutyric acid (aAib), γ-aminobutyric acid (yAbu), α-aminobutyric acid (aAbu), α-γ-aminobutyric acid (α-γ-Abu), 3-pyridylalanine (Pal), Isopropyl-α-N_{ε}-lysine (ILys), Napthyalanine (Nal), α-napthyalanine (α-Nal), β-napthyalanine (β-Nal), Acetyl-β-napthyalanine (Ac-β-napthyalanine), napthyalanine, N_{ε}-picoloyl-lysine (PicLys), 4-halo-Phenyl, 4-pyrolidylalanine, isonipecotic carboxylic acid (inip), β-amino acids, D-amino acids and isomers, analogues and derivatives thereof.

The immunogen in the treatment, e.g. the immunogenic peptide, can comprise one or more epitopes from a wide range of antigens of pathogens (infectious agents) and/or tumours. For example, the immunogen may comprise one or more epitopes from antigens from pathogens and infectious agents such as viruses, bacteria, fungi and protozoa. Some examples of pathogenic viruses causing infections or tumours from which epitopes from antigens may be derived include: hepatitis (A, B, or C), herpes virus (e.g., VZV, HSV-I, HAV-6, HSV-II, and CMV, Epstein Barr virus), adenovirus, SV40 virus (causing mesothelioma), influenza virus (as herein defined above), flaviviruses, ebola virus, echovirus, rhinovirus, coxsackie virus, coronavirus, respiratory syncytial virus (RSV), mumps virus, rotavirus, measles virus, rubella virus, parvovirus, vaccinia virus, HTLV virus, dengue virus, molluscum virus, poliovirus, rabies virus, JC virus, arboviral encephalitis virus, and human immunodeficiency virus (HIV virus; e.g., type I and II), human papilloma virus (HPV), more in particular of the high tumour risk types of HPV, comprising HPV-16, 18, 31, 33, 35, 39, 45, 51, 52, 56, 58, 59, and 68 types. Some examples of pathogenic bacteria causing infections from which epitopes from antigens may be derived include: *Listeria, Escherichia, Chlamydia, Rickettsial* bacteria, *Mycobacteria, Staphylococci, Streptocci, Pneumonococci, Meningococci, Gonococci, Klebsiella, Proteats, Serratia, Pseudomonas, Legionella, Diphtheria, Salmonella, Bacilli, Bordetella,* bacteria causing Cholera, Tetanus, Botulism, Anthrax, Plague, Leptospirosis, Whooping cough and Lymes disease. Some examples of pathogenic fungi causing infections from which epitopes from antigens may be derived include: *Candida (e.g., albicans, krusei, glabrata, tropicalis*)*, Cryptococcus neoformans, Aspergillus (e.g., fumigatus, niger*)*,* fungi of the genus *Mucorales* (*Mucor, Absidia, Rhizopus*)*, Sporothrix schenkii, Blastomyces dermatitidis, Paracoccidioides brasiliensis, Coccidioides immitis* and *Histoplasma capsulatum.* Some examples of pathogenic parasites causing infections from which epitopes from antigens may be derived include: *Entamoeba histolytica, Balantidium coli, Naegleria, Fowleri, Acanthamoeba sp., Giardia lambia, Cryptosporidium sp., Pneumocystis carinii, Plasmodium vivax, Babesia microti, Trypanosoma brucei, Trypanosoma cruzi, Leishmania donovani, Toxoplasma gondii* and *Plasmodium falciparis.*

In addition, the immunogen in the treatment , e.g. the immunogenic peptide, can comprise one or more epitopes from a wide range of tumor antigens, including e.g. MAGE, BAGE, RAGE, GAGE, SSX-2, NY-ESO-1, CT-antigen, CEA, PSA, p53, XAGE and PRAME but also virally induced malignancies, comprising Human papilloma virus (HPV), Kaposi sarcoma herpes virus (KSHV), Epstein Bar virus induced lymphoma's (EBV). Other examples of tumour antigens from which epitopes for use in the present invention may be derived are various ubiquitously expressed self-antigens that are known to be associated with cancer, which include e.g. p53, MDM-2, HDM2 and other proteins playing a role in p53 pathway, molecules such as survivin, telomerase, cytochrome P450 isoform 1B1, Her-2/neu, and CD19 and all so-called house hold proteins. Cancers that may be treated in accordance with the present invention are selected among the following list: lung, colon, esophagus, ovary, pancreas, skin, gastric, head and neck, bladder, sarcoma, prostate, hepatocellular, brain, adrenal, breast, endometrial, mesothelioma, renal, thyroid, hematological, carcinoid, melanoma, parathyroid, cervix, neuroblastoma, Wilms, testes, pituitary and pheochromocytoma cancers.

Preferably in the treatment, the immunogen, e.g. the immunogenic peptide, comprises one or more epitopes from a pathogen that are conserved among different strains of the pathogen. Surface exposed antigens of pathogens tend to undergo changes due to antigenic shifts and drifts, which can alter the antibody binding sites. These antigenic changes impair the neutralizing ability of antibodies induced by vaccines, rendering these vaccines ineffective. It is therefore preferred that the immunogen, e.g. the immunogenic peptide, comprises one or more epitopes from a pathogen that are conserved among different strains of the pathogen and that are less prone to antigenic shifts and drifts. Preferably these conserved epitopes are T-cell epitopes, i.e. at least one of a CTL epitope and a Th epitope, of which CTL epitopes are preferred.

A conserved epitope is herein understood as an epitope the amino acid sequence of which is at least 80, 81, 82, 83, 84, 85, 86, 88, 89, 90, 91, 92, 93, 94, 95, 96, 97, 98, 99 or 100% identical in at least 2, 5, 10, 20, 50 or 100 different strains of the pathogen, These different strains of the pathogen preferably are strains (or isolates) isolated from different subjects, preferably at different geographical locations and/or at different times, e.g. different annual or seasonal strains. Preferably, different strains of the pathogen are strains that differ in surface exposed antigens, more preferably different strains of the pathogen are strains of which the surface exposed antigens can be serologically and/or antigenically distinguished. Examples of two different strains include e.g. two influenza strains that differ in at least one of hemagglutinin and neuraminidase subtypes.

A conserved epitope is thus preferably an epitope that is not exposed at the surface of the pathogen, such as a virus. Preferably therefore, a conserved epitope is not a surface exposed of an influenza hemagglutinin and/or neuraminidase. Rather, preferably the conserved epitope is an non-surface exposed epitope, e.g. an epitope that is located in a viral protein present in the interior of the virus's virion, such as e.g. nucleoprotein and non-structural proteins such as polymerases. A preferred conserved epitope of an influenza virus is therefore an epitope located in an influenza protein selected from the group consisting of the matrix protein (M1), the membrane protein (M2), one RNA polymerase complex proteins (PB1, PB2 and PA) and nucleoprotein (NP).

Specific examples of immunogenic peptides, including conserved epitopes, are the synthetic peptides as specified in the Examples and sequence listings herein.

In a further aspect, the invention relates to a pharmaceutical composition. The pharmaceutical composition of the invention comprise a WIV as defined herein above and an immunogen as herein defined above. It is understood herein that the WIV and the immunogen are at least two separate entities that are added to each other in the preparation of the composition. Compositions comprising only the WIV without an externally added immunogen are preferably excluded from the invention.

The composition further preferably comprises a pharmaceutically acceptable carrier, medium or delivery vehicle as are conventionally known in the art (see e.g. "Handbook of Pharmaceutical Excipients", Rowe et al eds. 7th edition, 2012, Pharmaceutical Press, London). Pharmaceutically acceptable stabilizing agents, osmotic agents, buffering agents, dispersing agents, and the like may also be incorporated into the pharmaceutical compositions. The preferred form depends on the intended mode of administration and therapeutic application. The pharmaceutical carrier can be any compatible, non-toxic substance suitable to deliver the active ingredients, i.e. the WIV and the immunogen to the patient.

Pharmaceutically acceptable carriers for parenteral delivery are exemplified by sterile buffered 0.9% NaCl or 5% glucose optionally supplemented with a 20% albumin. Alternatively, the WIV and immunogen can be dissolved in Phosphate buffer saline (PBS). Preparations for parenteral administration must be sterile. The parenteral route for administration of the WIV and the immunogen is in accord with known methods, e.g. injection or infusion by intravenous, intraperitoneal, intramuscular, intraarterial or intralesional routes. A typical pharmaceutical composition for intramuscular injection would be made up to contain, for example, 1 - 10 ml of phosphate buffered saline comprising the above-indicated dosages of WIV and immunogenic peptide. Pharmaceutically acceptable carriers for intranasal delivery are exemplified by water, buffered saline solutions, glycerin, polysorbate 20, cremophor EL, and an aqueous mixture of caprylic/capric glyceride, and may be buffered to provide a neutral pH environment. For oral administration, the active ingredient can be administered in liquid dosage forms, such as elixirs, syrups, and suspensions. Liquid dosage forms for oral administration can contain colouring and flavouring to increase patient acceptance. Methods for preparing parenterally, orally or intranasally administrable compositions are well known in the art and described in more detail in various sources, including, for example, "Remington: The Science and Practice of Pharmacy" (Ed. Allen, L. V. 22nd edition, 2012, www.pharmpress.com).

In a further aspect, the invention provides a kit of parts comprising a WIV as defined herein above and an immunogen as herein defined above. The kit of parts preferably comprises a first container (e.g. a vial) comprising the WIV and a second container (e.g. a vial) and optionally further containers (vials) comprising one or more of the immunogens as defined above. Preferably, the first container comprises a pharmaceutical compositions for the WIV and the second (and further) container(s) comprise one or more of the immunogens, which pharmaceutical compositions are otherwise as described above. Preferably, the kit of parts is for use in a treatment as herein described above.

In yet a further aspect, the invention relates to a method for vaccination against, or for prophylaxis or therapy of an infectious disease or tumor by administration of a therapeutically or prophylactically effective amount of (a pharmaceutical composition comprising) a WIV as defined herein above and an immunogen as defined herein above, to a subject in need of prophylaxis or therapy. The invention also relates to a WIV as defined herein above and an immunogen as defined herein above for use as a medicament, preferably a medicament for vaccination against, or for prophylaxis or therapy of an infectious disease or tumor.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

### Description of the figures

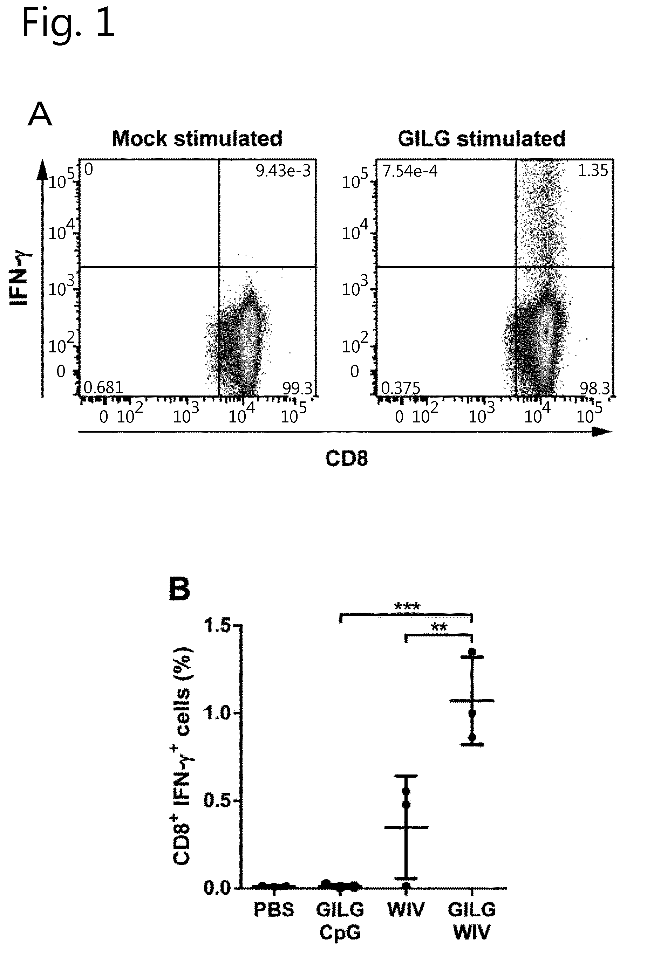

Figure 1. WIV acts as an adjuvant for peptide antigens. (A) Flow cytometry plot displaying specificity of IFN-γ⁺ CD8⁺ T-cells in splenocytes from mice immunized with GILG adjuvanated with WIV. (B) HLA-A2 transgenic mice were vaccinated twice with the shown formulations. Splenocytes restimulated with GILG peptide were analyzed for IFN-γ⁺ CD8⁺ T-cells with flow cytometry. Data is represented as mean ± SD, n=3; **p<0.01, ***p<0.001.

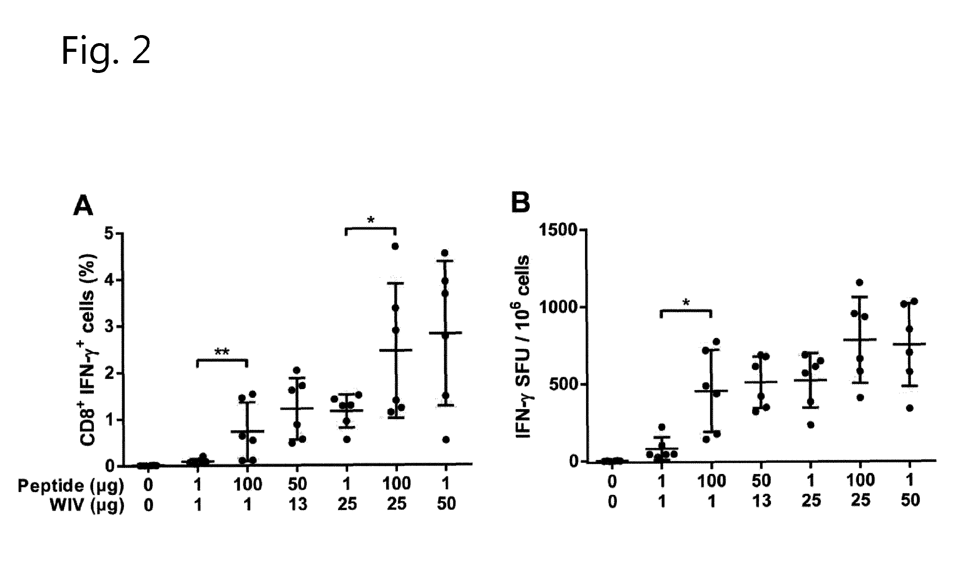

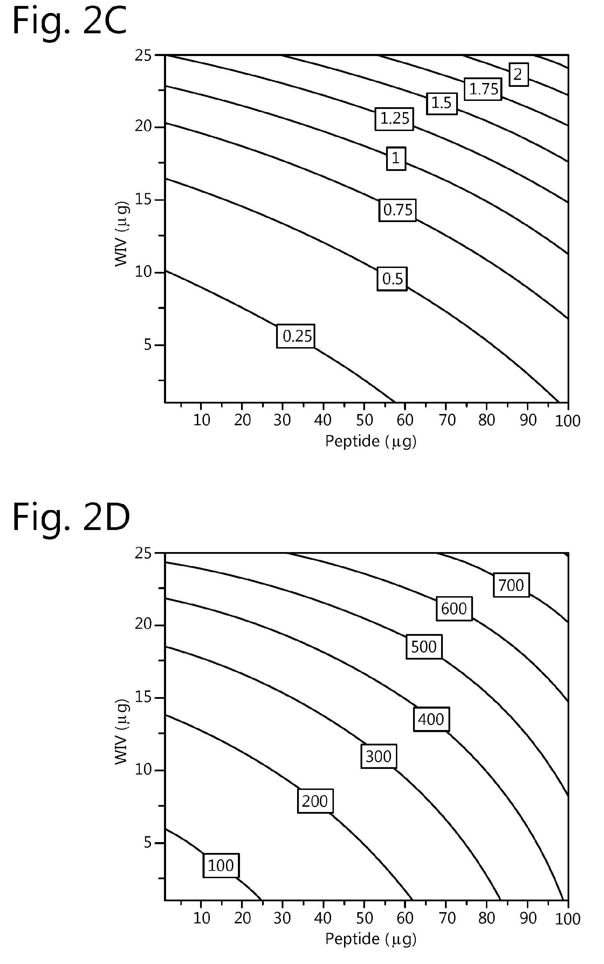

Figure 2. Dose-finding study of WIV and peptide by DoE. HLA-A2 transgenic BL/6 mice were vaccinated two times with different doses of M1₅₈₋₆₆ peptide and WIV. Splenocytes restimulated with peptide were analyzed for peptide-specific CD8⁺ T-cells with flow cytometry (A) and ELISpot (B). Prediction contour plots obtained by DoE visualize the interaction between peptide antigen and WIV for the flow cytometry (C) and ELISpot (D) responses. The predicted responses are displayed in the white boxes. Data is presented as mean ± SD, n=6; *p<0.05, **p<0.01.

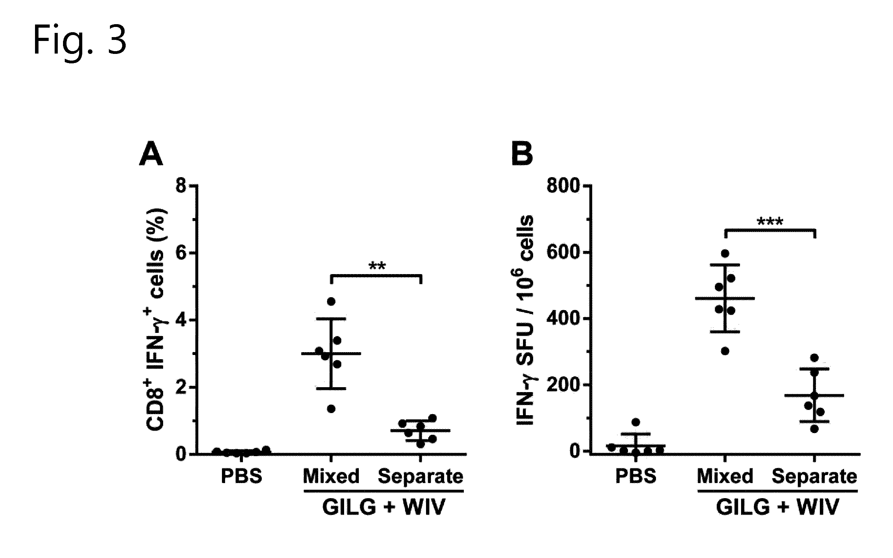

Figure 3. Effect of co-localization on WIV adjuvanticity. HLA-A2 transgenic BL/6 mice were vaccinated twice with 100 µg GILG peptide and 25 µg WIV either in one single flank or separate flanks. Splenocytes were restimulated with GILG peptide and analyzed with flow cytometry (A) or ELISpot (B). Data is presented as mean ± SD, n=6; **p<0.01, ***p<0.001.

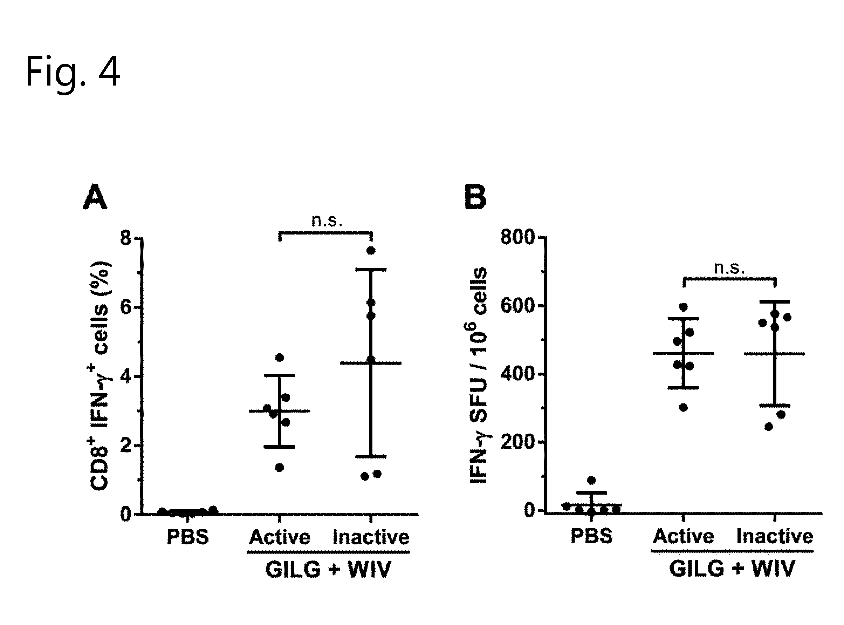

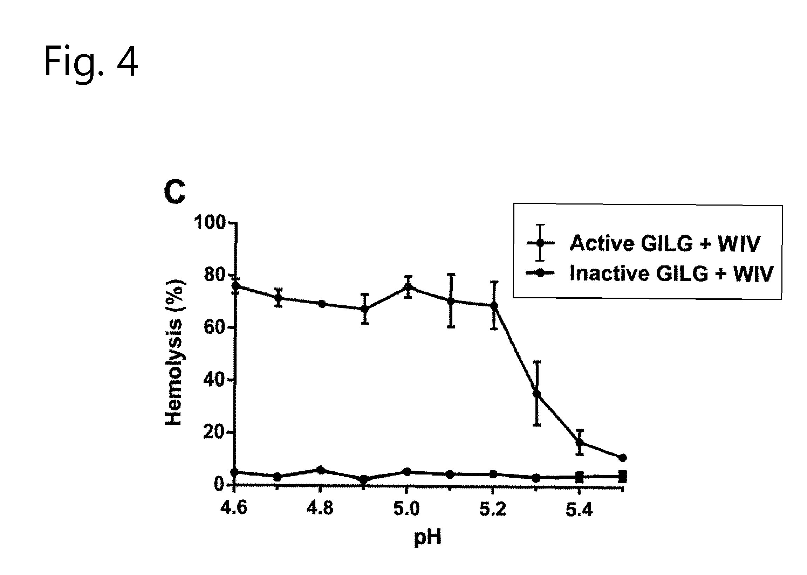

Figure 4. Effect of membrane fusion activity on WIV adjuvanticity. HLA-A2 transgenic BL/6 mice were vaccinated twice with 100 µg GILG peptide and 25 µg fusion-active or fusion-inactive WIV. Splenocytes were restimulated with GILG peptide and analyzed with flow cytometry (A) or ELISpot (B). Fusion activity of active and inactive WIV-GILG formulations was determined by hemolysis assay (C). Immunogenicity data is presented as mean ± SD n=6; n.s.=not significant. Hemolysis data is presented as mean ± SD n=3.

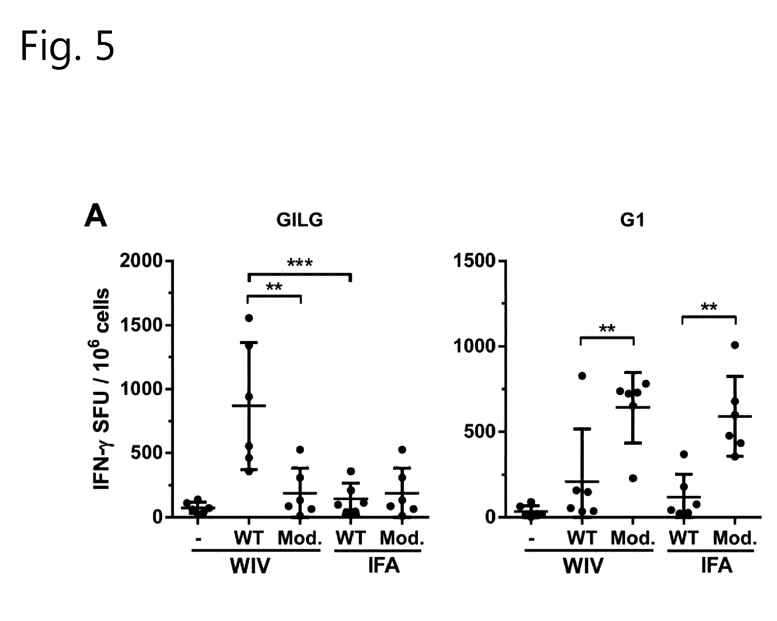

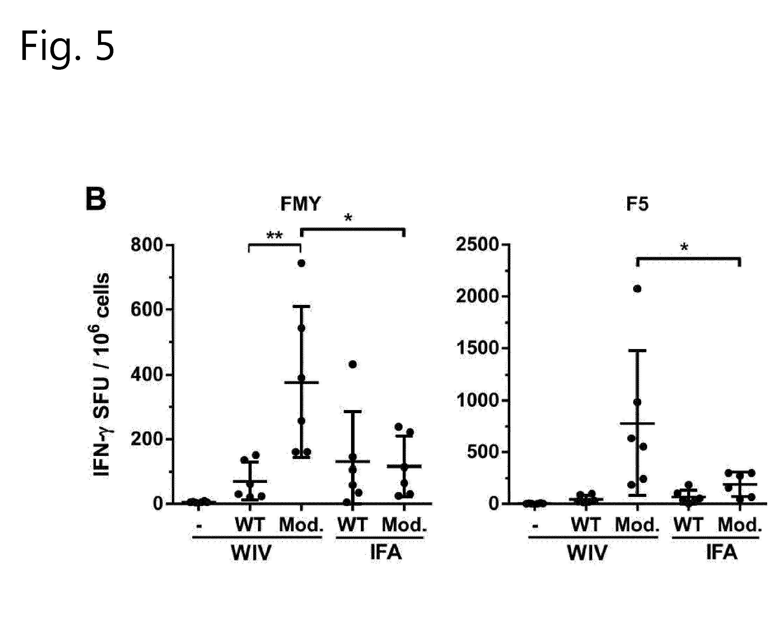

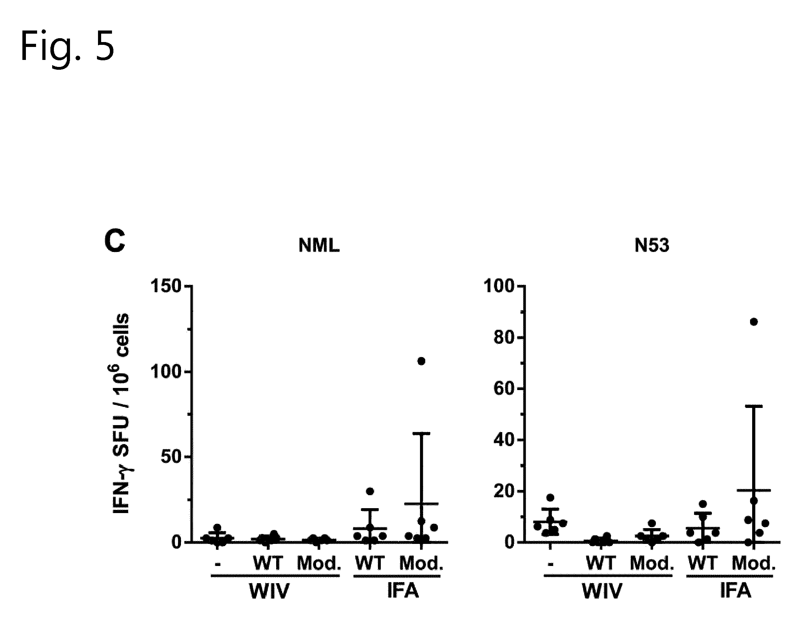

Figure 5. T-cell responses against wild-type and modified peptides adjuvanated with WIV. HLA-A2 transgenic BL/6 mice were vaccinated twice with peptide pools containing wild-type (WT) peptides (GILG, FMY and NML) or modified (mod.) peptides (G1, F5 and N53) adjuvanated with WIV or IFA. Specific T-cell responses induced by the peptide pools towards either GILG or G1 (A), FMY or F5 (B), NML or N53 (C) were determined for all groups. IFN-γ spot-forming units were determined with ELISpot. Data is presented as mean ± SD n=6; *p<0.05, **p<0.01, ***p<0.001.

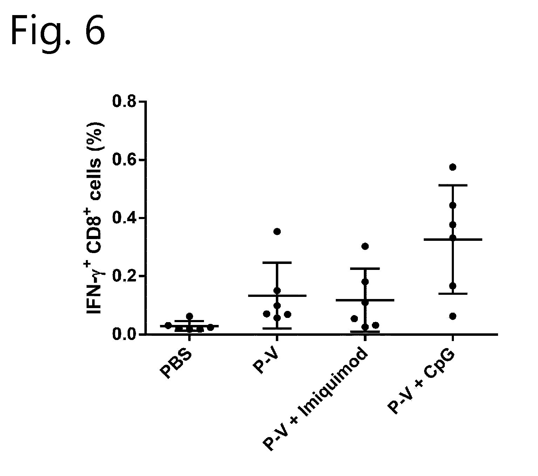

Figure 6. HLA-A2 BL/6 mice were immunized with peptide-loaded virosomes (P-V) and the adjuvants Imiquimod or CpG. Peptide-specific T-cells were quantified using flow cytometry. N=6.

### Examples

### 1. Materials and methods

### 1.1 Formulation of vaccines

Influenza A/PR8/34 virus was propagated on fertilized eggs and inactivated with β-propiolactone on a pilot scale as described before [12], which yielded PR8 WIV bulk vaccine. To study the effect of fusion activity on the immune response, WIV was fusion-inactivated by lowering the buffer pH to 4.5 with a pretitrated volume of 1 M HC1, and subsequently incubated at 37°C for 15 min. Afterwards, the sample was brought to physiological pH by dialyzing overnight against PBS pH 7.2. Membrane fusion capacity was subsequently determined by a hemolysis assay as described previously [8].

The HLA-A2 restricted influenza GILGFVFTL (GILG, M1₅₈₋₆₆; SEQ ID NO: 1), FMYSDFHFI (FMY, PA₄₆₋₅₄; SEQ ID NO: 2) and NMLSTVLGV (NML, PB1₄₁₃₋₄₂₁; SEQ ID NO: 3) peptides were synthesized using standard methods.

Influenza PR8 WIV and peptide antigens were formulated in PBS pH 7.2 (Life Technologies) at various concentrations. When mentioned, 50 µg of CpG ODN 1826 (Invivogen) or 50% (v/v) Incomplete Freund's adjuvant (IFA, Sigma-Aldrich) was added to the formulation.

### 1.2 Animal studies

Animal studies were conducted according to the guidelines provided by the Dutch Animal Protection Act, and were approved by the Committee for Animal Experimentation (DEC) of the National Institute for Public Health and the Environment (RIVM). Eight- to ten-week-old female transgenic HLA-A2.1 C57BL/6 mice (Jackson Laboratory, maintained in-house) were used in all studies.

In the proof-of-principle study, mice (three per group) received immunizations subcutaneously (s.c.) in the flank at day 0 and 21 under isoflurane anesthesia, containing either PBS, 50 µg WIV or 1 µg GILG peptide adjuvanted with either 50 µg WIV or 50 µg CpG. Animals were sacrificed by cervical dislocation and bleeding under anesthesia at day 35.

For the dose finding study, a DoE approach was used (as described below). The selected formulations consisting of various doses of WIV and GILG peptide (shown in Table 1) were administered s.c. in the flank of mice (six per group) under isoflurane anesthesia at day 0 and 21. Animals were sacrificed by cervical dislocation and bleeding under anesthesia at day 35.

To study the effect of adjuvant co-localization, mice (six per group) were immunized at day 0 and 21 either s.c. in one flank with PBS or 100 µg GILG peptide adjuvanted with 25 µg WIV, or s.c. in separate flanks with 100 µg GILG peptide in one flank and 25 µg WIV adjuvant in the opposite flank. Animals were sacrificed by cervical dislocation and bleeding under anesthesia at day 35.

**Table 1.**

| **No.** | **WIV** | **Peptide** | **No.** | **WIV** | **Peptide** | **No.** | **WIV** | **Peptide** |
|---|---|---|---|---|---|---|---|---|
| **1** | 1 | 1 | **11** | 25 | 1 | **21** | 1 | 100 |
| **2** | 1 | 1 | **12** | 25 | 1 | **22** | 1 | 100 |
| **3** | 1 | 1 | **13** | 13 | 50.5 | **23** | 1 | 100 |
| **4** | 1 | 1 | **14** | 13 | 50.5 | **24** | 1 | 100 |
| **5** | 1 | 1 | **15** | 13 | 50.5 | **25** | 25 | 100 |
| **6** | 1 | 1 | **16** | 13 | 50.5 | **26** | 25 | 100 |
| **7** | 25 | 1 | **17** | 13 | 50.5 | **27** | 25 | 100 |
| **8** | 25 | 1 | **18** | 13 | 50.5 | **28** | 25 | 100 |
| **9** | 25 | 1 | **19** | 1 | 100 | **29** | 25 | 100 |
| **10** | 25 | 1 | **20** | 1 | 100 | **30** | 25 | 100 |

The effect of membrane fusion activity was assessed by immunizing mice (six per group) s.c. in the flank at day 0 and 21 with 100 µg GILG peptide adjuvanted with either 25 µg of fusion-active WIV or fusion-inactive WIV. Animals were sacrificed by cervical dislocation and bleeding under anesthesia at day 35.

The adjuvant effect of WIV on a mix of multiple peptides was assessed with either a WT peptide pool (GILG, FMY and NML; 100 µg each) or a modified peptide pool (G1, F5 and N53; 100 µg each). Mice (six per group) received an s.c. immunization in the flank at day 0 and 21 containing either PBS, WT peptide pool adjuvanted with 5 µg WIV or IFA, modified peptide pool adjuvanted with 5 µg WIV or IFA, or only 5 µg WIV. Animals were sacrificed by cervical dislocation and bleeding under anesthesia at day 35.

### 1.3 Dose finding by Design of Experiments

A initial dose-response study was performed by a Design of Experiments approach in order to detect potential interactions and effects between the GILG peptide antigen dose and WIV adjuvant dose on the induction of GILG-specific T-cell responses in vivo. A Full Factorial Design was created using MODDE 10.0.0 (Umetrics AB). Results from both flow cytometry and ELISpot methods were selected as response parameters. The limits of the doses ranged from 1-100 µg GILG peptide and 1-25 µg WIV. This resulted in a design with seven formulations including three center points. To accommodate for the high variability in animal experiments, it was chosen to administer each formulation to six mice, resulting in a design as shown in Table 1. The models were fitted using partial least squares and subsequently optimized by deleting non-significant terms [13], until the model performance parameters goodness of fit (R²), goodness of prediction (Q²), validity and reproducibility were at their highest.

### 1.4 Intracellular staining

T-cell populations were assessed by flow cytometry. In short, single-cell suspensions of spleens were plated at a concentration of 2*10⁶ cells in a 48-well plate in RPMI medium (Life Technologies) with 10% Hyclone fetal calf serum (FCS, Thermo Scientific), and stimulated overnight with either medium, 50 ng M1₅₈₋₆₆ peptide or PR8 WIV. Cytokine transport was inhibited by incubating with Golgi-plug (BD Biosciences) for 4 hours. Cells were subsequently stained with anti-mouse CD8-FITC (BD Biosciences), anti-mouse CD4-PE (BD Biosciences) and Live-dead-Aqua (Invitrogen). Next, cells were fixated with fixation/permeabilization buffer (BD Biosciences) and washed with permeabilization wash buffer (BD Biosciences). Finally, cells were stained intracellular with anti-mouse IFN-γ-APC (BD Biosciences), and analyzed on a FACS Canto II flow cytometer (BD Biosciences). Acquired data was analyzed with FlowJo version 10 (TreeStar Inc.).

### 1.5 Enzyme linked immunosorbent spot assay (ELISpot)

An ELISpot assay was used to determine IFN-γ spot-forming units in restimulated splenocytes. 96-wells Multiscreen PVDF filter plates (Millipore) were activated by adding 25 µL 70% ethanol for 2 min, and subsequently washed three times with PBS. Plates were coated overnight with anti-mouse IFN-γ antibodies (U-Cytech) at 4°C. Next, filter plates were washed three times and blocked with 5% Hyclone fetal calf serum (FCS, Thermo Scientific) for 1 hour at 37°C. Subsequently, 4*10⁵ isolated splenocytes resuspended in IMDM medium, 5% FCS were added to each well with or without 50 ng relevant peptide, and incubated overnight at 37°C. After overnight stimulation, filter plates were washed five times and IFN-γ was detected using biotinylated anti-mouse antibodies (U-Cytech) and 100 µL BCIP/NBT reagent (Thermo Scientific) per well. Spots were allowed to develop for 15 min after which the plates were thoroughly washed with tap water. Spots were counted using an A.EL.VIS ELISpot reader (Aelvis). The number of IFN-γ producing cells in antigen-stimulated splenocytes was obtained after background correction (subtracting number of spots produced by splenocytes incubated with medium).

### 1.6 Hemolysis assay

Virosome fusion activity was determined by using a hemolysis assay as described previously [14]. Formulations were mixed with human blood erythrocytes and 0.1M 2-(N-morpholino)ethanesulfonic acid (MES) buffer with pH's ranging from 4.5 to 5.5, and incubated at 37°C for 30 min. The released hemoglobin was quantified in the supernatant after centrifugation by reading absorbance at 540 nm using a Synergy Mx reader (Biotek). Hemoglobin release of erythrocytes mixed with water was set as maximal hemolysis (100%).

### 1.7 Statistics

Results were statistically analyzed with a one-way ANOVA followed by a Tukey-post test for multiple comparisons. All statistical analyses were performed using GraphPad Prism 6.04 for Windows (GraphPad Software Inc.).

### 2. Results and discussion

### 2.1 Proof-of-principle of WIV as an adjuvant

The adjuvant effect of WIV for GILG peptide was assessed in HLA-A2.1 transgenic mice. They received two vaccinations of either peptide adjuvanted with CpG, peptide adjuvanted with WIV or WIV alone. Splenocytes restimulated with GILG peptide were analyzed for peptide-specific T cells by flow cytometry. The specificity of CD8⁺ IFN-γ⁺ T cells was determined by comparing peptide-stimulated splenocytes with mock-stimulated splenocytes (Figure 1A).

As expected, 100 µg GILG peptide adjuvanted solely with 50 µg CpG did not induce any peptide-specific CTL response in mice (Figure 1B). This can be attributed to a number of factors, such as the absence of CD4⁺ helper epitopes, and the lack of delivery of antigen and adjuvant, both of which are crucial for the immunogenicity of short peptide antigens [4]. In contrast, only 1 µg GILG peptide antigen adjuvanted with 50 µg WIV induced peptide-specific responses in mice. Mice that received only WIV also showed considerable T cell responses, which was attributed to the high dose of WIV as described earlier [15, 16]. The GILG epitope is indeed present in PR8 WIV, which explains the induction of GILG-specific T cells by WIV at high concentrations. Furthermore, WIV might still act as an adjuvant for peptides at lower doses. Thus, in order to maximize peptide-specific T cell responses with minimal use of WIV, a dose-finding study was conducted.

### 2.2 Dose-finding and interaction study between GILG peptide and WIV using design of experiments

To investigate which concentrations of both WIV and peptide were still able to induce a peptide-specific T cell response a dose-finding study of both WIV and peptide was conducted by using a DOE approach. DOE approaches are commonly used for the optimization of (bio)pharmaceutical formulations [17]. However, there are currently no reports that utilized a DoE approach to assess the effect of formulation parameters on in vivo responses, such as cellular immune responses. A full factorial design was implemented by varying the peptide antigen dose from 1-100 µg, and the WIV adjuvant dose from 1-25 µg.

The formulations, containing a variety of GILG peptide and WIV doses, were tested for their ability to induce GILG-specific T cell responses in HLA-A2.1 transgenic mice (Figure 2A and 2B). A combination of 1 µg GILG peptide and 1 µg WIV was unable to induce CTL responses. However, when the GILG dose was increased to 100 µg, a significant increase of GILG-specific T cells was observed. This effect was also observed when the peptide dose was increased from 1 to 100 µg combined with a dose of 25 µg WIV. These results indicate that WIV is still able to boost the immune responses towards GILG peptide at concentrations as low as 1 µg WIV when combined with a high dose (100 µg) of peptide antigen.

Association between the GILG peptide and the WIV particles may be a contributing factor to the immunogenicity of peptide antigen. Thus, the association between the peptide and WIV was determined (Table 2). Only low amounts of GILG peptide (1 µg) mixed with relatively high amounts of WIV (25 or 50 µg) showed some association. At higher peptide concentrations, association with WIV was negligible, which can be explained by the high molar abundance of GILG peptide compared to WIV or a low affinity between the two. In general, it can be concluded that association of the peptide to WIV did not have a significant influence on the immunogenicity, contrarily to other delivery systems such as liposomes or virosomes [8, 18].

**Table 2. Association between GILG peptide and WIV at different concentrations. The fraction of unassociated peptide was determined in the supernatant by mass spectrometry. Data represents mean ± SD, n=3.**

| **Peptide (µg)** | **WIV (µg)** | **Unassociated peptide (%)** |
|---|---|---|
| 1 | 1 | 112 ± 10 |
| 100 | 1 | 111 ± 6 |
| 50 | 13 | 96 ± 5 |
| 1 | 25 | 77 ± 8 |
| 100 | 25 | 92 ± 20 |
| 1 | 50 | 87 ± 9 |

To assess the synergistic effect between the peptide antigen and the WIV adjuvant, a partial least squared (PLS) regression model was fitted for the T cell responses. Valid models were obtained for both flow cytometry (R²=0.706, Q²=0.633) and ELISpot (R²=0.712, Q²=0.629) responses, and model prediction contour plots were generated (Figure 2C and 2D). The contour plots illustrate that addition of WIV is essential for the peptide antigen to become immunogenic. Furthermore, the model indicates that theoretically the optimum of T cell responses has not been reached yet; however, the dose ranges used in this DoE model for both GILG peptide and WIV are at their maximum concerning peptide solubility and feasible WIV dose for human use, respectively.

The use of the DoE approach enabled us to illustrate the synergy between antigen and adjuvant, and to predict their effect on the cellular immune responses in vivo. The use of DoE in preclinical animal studies is difficult, due to the multiple factors, such as biological variability between animals and T cell assay variability, which can cause variability in each animal study. Nonetheless, the use of DoE provides valuable insight in the effect of antigen and adjuvant dose on the immune response in mice, and could be implemented in future vaccine development.

### 2.3 Involvement of WIV-peptide co-localization on immunogenicity

The viral ssRNA present in WIV is a TLR7 agonist, and likely contributes to the observed immunostimulating effect of WIV [9]. For most adjuvants, including TLR ligands, co-localization with the antigen is necessary to provide local immunostimulatory signals.

In the model contour plots, it is predicted that a dose of 25 µg WIV combined with 100 µg GILG peptide is able to induce the highest peptide-specific T cell responses. Therefore, 25 µg WIV and 100 µg peptide was selected as the formulation to be used in mechanistic studies. To investigate the importance of co-localization, we administered 100 µg GILG peptide and 25 µg WIV s.c. at separate flanks, each draining to a different lymph node. When the peptide and WIV were administered separately at different sites, a significant decrease of peptide-specific T cell response was observed (Figures 3). The observed response after separate vaccination is probably caused by the WIV only. It is likely that co-localization of WIV and GILG peptide in the endosomal compartment of APCs is required to benefit from the co-stimulatory adjuvant signal provided by the viral ssRNA [19]. Moreover, a recent study suggested that particulate delivery of a TLR7 agonist can improve its immunostimulatory effect due to efficient delivery to the endosomal compartment [20], where TLR7 is located. WIV can deliver its own viral ssRNA in a similar manner, which might explain the immunostimulatory potential of WIV.

### 2.4 Involvement of WIV membrane fusion activity on immunogenicity

Aside from co-localization, the role of membrane fusion activity of WIV was investigated. Fusion activity was shown to be important for the induction of cross-reactive T cell responses by WIV [21]. Furthermore, other nearby molecules, such as the peptide antigen in our WIV-adjuvanted vaccine, can escape the endosomal compartment during membrane fusion of WIV with the endosomal membrane [22]. Fusion activity might thus play a role in the adjuvanticity of WIV. Surprisingly, mice vaccinated with fusion-inactivated WIV mixed with GILG peptide still produced high amounts of peptide-specific T cells, comparable to those in mice receiving fusion-active WIV with peptide (Figures 4A and 5B). A hemolysis assay confirmed the loss of pH-dependent fusion activity of WIV (Figure 4C). The current results indicate that fusion activity of WIV is not important for the induction of T cell responses against peptide antigens. The immunogenicity of antigens located inside the WIV particle itself might be compromised by fusion inactivation as shown before [21], but in the current study the admixed GILG peptide apparently was taken up and processed correctly by APCs regardless of WIV fusion activity. This indicates that WIV is a robust adjuvant that retains its function even after loss of fusogenicity.

### 2.5 Adjuvation of multiple peptides by WIV

To investigate whether WIV also acts as an adjuvant for multiple peptides, a peptide pool of GILG and two additional human HLA-A2.1-restricted influenza epitopes, FMY and NML, was studied in combination with WIV. In addition, we selected three modified peptides to be combined with WIV, being G1, F5 and N53, which are CPLs derived from the three aforementioned WT peptide epitopes. Modification of WT peptides with non-proteogenic amino acids has previously shown to increase binding affinity with the MHC-I molecules, which might result in increased T cell responses [11]. Since the selected epitopes are also present in WIV, a reduced WIV dose (5 µg) was chosen from the previously established prediction model. At this concentration, it was predicted that WIV still had an immunostimulating effect, while bringing the inherent T cell response generated by WIV itself to a minimum. Mice were vaccinated with either WT or modified peptide pools adjuvanted with WIV. As a control, peptide pools adjuvanted with IFA were included to compare the adjuvanticity of WIV to that of IFA.

The individual peptides in both WT and modified pools did not show significant association with the WIV particles, similar to the previous observations with the GILG peptide alone in this study (data not shown). Thus, it is unlikely that differences in induced immune responses by the peptide vaccines are caused by differences in association between peptide and WIV.

As seen previously in this study, the GILG peptide in the peptide pool was able to induce GILG-specific T cell responses after immunostimulation with WIV (Figure 5A). In contrast, IFA adjuvanted GILG peptide induced significantly lower T cell responses. The modified G1 peptide however was unable to induce potent GILG-specific responses, regardless of adjuvant. The G1 peptide adjuvanted with either WIV or IFA did induce a G1-specific T cell response, indicating that while the modified peptide was immunogenic in combination with an adjuvant, it failed to induce responses that reacted with the WT analog.

The WT FMY peptide was able to induce modest FMY-specific T cell responses in combination with either WIV or IFA (Figure 5B). Interestingly, the modified F5 peptide was able to induce significantly higher FMY-specific responses compared to the WT FMY peptide when adjuvanted with WIV. F5 peptide adjuvanted with IFA did not show such an increase, indicating that WIV is a more potent adjuvant than IFA for the F5 peptide. This difference was also observed with the F5-specific responses; F5 peptide induced significantly higher F5-specific T cell responses when adjuvanted with WIV than with IFA.

The subdominant NML peptide and the modified N53 were unable to induce any significant T cell responses, regardless of adjuvant (Figure 5C). IFA-adjuvanted peptides showed incidental T cell responses in some animals, suggesting that IFA is a slightly better adjuvant than WIV for this specific peptide. It is unclear why WIV was not effective with NML and N53 peptides, while IFA-adjuvanted NML and N53 managed to induce a response in a few animals. However, since responses induced by IFA-adjuvanted NML and N53 peptides were not consistent in all animals, there was no significant difference between IFA- and WIV-adjuvanted groups.

These data indicate that WIV is a potent adjuvant for short peptides, both in WT or modified form. Other approaches such as peptide-lipid conjugates [23], liposomes [7], virosomes and nanoparticles have been used previously to increase the immunogenicity of short peptides [8, 24], but require multiple formulation steps and might not be suitable for every peptide due to differences in physicochemical attributes. In contrast, WIV can be readily mixed with peptide antigens, which is a simple process to scale up. Furthermore, WIV is already licensed and used for decades as an influenza vaccine, and recent studies show an excellent safety profile [25]. With this prior knowledge on safety and tolerability, it should be feasible to include WIV in any prospective vaccine as an adjuvant.

### 2.6 Conclusion

While it is known that WIV possesses an innate adjuvant capacity, so far it has never been used as an adjuvant for peptide antigens. We showed that WIV is capable of effectively increasing the T cell response against GILG and FMY influenza peptides in HLA-A2.1 transgenic mice. Co-localization of antigen and adjuvant were necessary to induce a potent T cell response, but the membrane fusion capacity of WIV was not important for the immunogenicity of the formulation. Furthermore, we showed that WIV was also able to immunostimulate non-natural, modified peptides effectively. Due to the ease of production of WIV and its long time safety track record, it is an excellent candidate adjuvant for low-immunogenic antigens that induce cellular responses.

### 3. Comparative Example

The adjuvant effect of WIV is most probably mediated by the presence of viral ssRNA in WIV, which is a natural TLR7 ligand. In a previous study, we compared the adjuvanticity of synthetic TLR7 ligand imiquimod to CpG-ODN, a TLR9 ligand, in combination with peptide-loaded virosomes, which previously were able to induce peptide-specific T-cells [20].

Figure 6 shows the results of the comparison of TLR agonists in combination with peptide-loaded virosomes (P-V). It is shown that imiquimod was unable to boost the immune responses against P-V. In contrast, CpG adjuvanted P-V performed considerably better. Our novel formulation of peptide antigens adjuvanted with WIV however, induces T-cell responses that are significantly higher than P-V+CpG formulations. Thus, the adjuvant effect of WIV is superior to the adjuvant effect of imiquimod, while both contain TLR7 ligands. We therefore conclude that an additional mechanism is responsible for the improved adjuvanticity of WIV as found by the inventors.

### References

1. Sridhar, S., et al., Cellular immune correlates of protection against symptomatic pandemic influenza. Nat Med, 2013. 19(10): p. 1305-12.
2. Foged, C., J. Hansen, and E.M. Agger, License to kill: Formulation requirements for optimal priming of CD8(+) CTL responses with particulate vaccine delivery systems. Eur J Pharm Sci, 2012. 45(4): p. 482-91.
3. Amorij, J.P., et al., Towards tailored vaccine delivery: needs, challenges and perspectives. J Control Release, 2012. 161(2): p. 363-76.
4. Rosendahl Huber, S., et al., T cell responses to viral infections - opportunities for Peptide vaccination. Front Immunol, 2014. 5: p. 171.
5. Arkema, A., et al., Induction of cytotoxic T lymphocyte activity by fusion-active peptide-containing virosomes. Vaccine, 2000. 18(14): p. 1327-33.
6. Ichihashi, T., et al., Cross-protective peptide vaccine against influenza A viruses developed in HLA-A*2402 human immunity model. PLoS One, 2011. 6(9): p. e24626.
7. Nagata, T., et al., Peptides coupled to the surface of a kind of liposome protect infection of influenza viruses. Vaccine, 2007. 25(26): p. 4914-21.
8. Soema, P.C., et al., Influenza T-cell Epitope-Loaded Virosomes Adjuvanted with CpG as a Potential Influenza Vaccine. Pharm Res, 2014.
9. Budimir, N., et al., Critical role of TLR7 signaling in the priming of cross-protective cytotoxic T lymphocyte responses by a whole inactivated influenza virus vaccine. PLoS One, 2013. 8(5): p. e63163.
10. Wiesel, M. and A. Oxenius, From crucial to negligible: functional CD8(+) T-cell responses and their dependence on CD4(+) T-cell help. Eur J Immunol, 2012. 42(5): p. 1080-8.
11. Hoppes, R., et al., Altered Peptide Ligands Revisited: Vaccine Design through Chemically Modified HLA-A2-Restricted T Cell Epitopes. J Immunol, 2014. 193(10): p. 4803-13.
12. Hendriks, J., et al., An international technology platform for influenza vaccines. Vaccine, 2011. 29 Suppl 1: p. A8-11.
13. Kraan, H., et al., Development of thermostable lyophilized inactivated polio vaccine. Pharm Res, 2014. 31(10): p. 2618-29.
14. Stegmann, T., et al., Functional reconstitution of influenza virus envelopes. EMBO J, 1987. 6(9): p. 2651-9.
15. Budimir, N., et al., The role of membrane fusion activity of a whole inactivated influenza virus vaccine in (re)activation of influenza-specific cytotoxic T lymphocytes. Vaccine, 2010. 28(52): p. 8280-7.
16. Bodewes, R., et al., Vaccination with whole inactivated virus vaccine affects the induction of heterosubtypic immunity against influenza virus A/H5N1 and immunodominance of virus-specific CD8+ T-cell responses in mice. J Gen Virol, 2010. 91(Pt 7): p. 1743-53.
17. van der Pol, L. and J.P. Amorij, Vaccine Development: History, Current Status, and Future Trends, in Biological Drug Products: Development and Strategies. 2014, John Wiley & Sons, Inc. p. 411-435.
18. Lund, J.M., et al., Recognition of single-stranded RNA viruses by Toll-like receptor 7. Proc Natl Acad Sci USA, 2004. 101(15): p. 5598-603.
19. Budimir, N., et al., Induction of heterosubtypic cross-protection against influenza by a whole inactivated virus vaccine: the role of viral membrane fusion activity. PLoS One, 2012. 7(1): p. e30898.
20. Soema, P. C., S. K. Rosendahl Huber, G. J. Willems, W. Jiskoot, G. F. Kersten and J. P. Amorij (2014). "Influenza T-cell Epitope-Loaded Virosomes Adjuvanted with CpG as a Potential Influenza Vaccine." Pharm Res.

## Claims

1. Whole inactivated influenza virus (WIV) for use as adjuvant in the prevention and/or treatment of a disease or a condition associated with an antigen, wherein the treatment comprises raising an immune response against the antigen by administration of the WIV and an immunogen comprising or encoding at least one epitope of the antigen, and wherein the WIV is a chemically inactivated WIV, preferably a WIV inactivated by at least one of formalin and β-propiolactone.

2. A WIV for a use according to claim 1, wherein the disease or a condition is an infectious disease and/or a tumour, wherein the immunogen induces an immune response against an antigen of an agent causing the infectious disease and/or an antigen of the tumour.

3. A WIV for a use according to claim 1 or 2, wherein the treatment comprises co-localized administration of the WIV and the immunogen, wherein preferably, the co-localized administration comprises administration at one or more sites of administration that drain to the same lymph node.

4. A WIV for a use according to claim 3, wherein the co-localized administration comprises the administration of a single composition comprising both the WIV and the immunogen.

5. A WIV for a use according to any one of the preceding claims, wherein the immune response is a cellular immune response, preferably a T-cell response comprising at least one of a CD8⁺ cytotoxic T-cell (CTL) response and a CD4⁺ T helper (Th) cell response.

6. A WIV for a use according to any one of the preceding claims, wherein the immunogen is selected from the group consisting of a protein, a peptide, a chemically modified protein or peptide, a protein or peptide comprising non-proteogenic amino acids, a polysaccharide, a whole bacterial cell, a membrane vesicle, an outer membrane vesicle, a virion, a virus-like particle, a nucleic acid and conjugates and combinations of thereof.

7. A WIV for a use according to claim 6, wherein the antigen is a proteinaceous antigen and the immunogen is a peptide comprising at least one epitope of the antigen.

8. A WIV for a use according to claim 7, wherein the peptide comprise at least one of an HLA class I restricted CTL epitope and an HLA class II restricted Th epitope.

9. A WIV for a use according to claim 8, wherein the peptide comprises a CTL epitope of a virus, which epitope is conserved between different strains of the virus, which epitope preferably is not exposed at the surface of the virus and wherein more preferably, the virus is influenza.

10. A WIV for a use according to claim 9, wherein the peptide comprises a conserved CTL epitope located in an influenza protein selected from the group consisting of the matrix protein (M1), the membrane protein (M2), one RNA polymerase complex proteins (PB1, PB2 and PA) and nucleoprotein (NP).

11. A WIV for a use according to any one of claims 6 - 10, wherein the peptide is at least one of: a) a peptide having a length of between 6 and 100 amino acids; and, b) a peptide comprising one or more non-proteogenic amino acids.

12. A WIV for a use according to any one of the preceding claims, wherein the WIV is a WIV vaccines, preferably a WIV vaccine comprising interpandemic annual or/and seasonal influenza strains.

13. A WIV for a use according to any one of the preceding claims, wherein the WIV is administered in a dosage of between 0.5 and 500 µg hemagglutinin per dose.

14. A pharmaceutical composition comprising a WIV as defined in any one of the preceding claims and an immunogen as defined in any one of the preceding claims, wherein preferably, the immunogen is present as an entity separate from the WIV.

15. A kit of parts comprising a first container comprising a WIV as defined in any one of the preceding claims and at least a second container comprising an immunogen as defined in any one of the preceding claims.
